# EUROPEAN PATENT APPLICATION

(11) **EP 2 573 560 A1**
(43) Date of publication of application: **27.03.2013**
(21) Application number: 12176204.1
(22) Date of filing: 12.07.2012
(51) Int. Cl.: G01N 33/543, B01L 3/00, G01N 33/493

(54) **Unitized point-of-care urine dipstick control device**

(30) Priority: 21.09.2011 US 201113239103; 13.02.2012 US 201213371716
(71) Applicant: Ban,, Monty Scott, Redondo Beach, CA 90278 (US)
(72) Inventor: Ban, Monty Scott, California, CA California 90278 (US); Popp, Nicholas Eugene, California, CA California 92009 (US)
(74) Representative: Lunt, Mark George Francis

(57) **Abstract**

A control device suitable for a urine reagent strip comprises an elongated package having front and rear walls, a top portion and a bottom portion, and an elongated chamber partially filled with test urine. An elongated chamber extends from the top portion to the bottom portion. At least a portion of one of the walls at the chamber is substantially transparent or translucent. The device preferably includes a flap of at least one quarter inch wide on one side of the chamber and a closed access section in the top portion. User information is on at least one of the walls.

## Description

This invention relates to urine testing devices; and, more particularly, a device for determining the performance of a urine dipstick to ensure accurate results from patient urine specimens.

### BACKGROUND

Urinalysis, also called a routine urine test, is used to check for abnormalities in urine. Urine is liquid waste that is made by the kidneys and stored in the bladder until it is eliminated by the body through urination. A number of diseases and conditions can result in abnormalities in the urine. These abnormalities may be detected through physical, chemical, and microscopic examination. Urinalysis may be performed as part of a regular physical examination to screen for early signs of disease.

Urinalysis was the very first laboratory test developed - the value of urine testing to diagnose medical conditions was established over 6000 years ago. There is evidence of visual testing of urine in the earliest civilizations. As advances in medicine are proved visual testing to be unreliable, chemical evaluation of the urine replaced this method as a more accurate means of diagnosis.

Further understanding of the structure of the body (anatomy) and of the physical and chemical processes involved in organ function (physiology), as well as the invention and development of the microscope, led to additional advances in urine testing as a diagnostic tool. Urinalysis was first introduced as part of the routine physical examination in 1827, and this test remains a valuable method of diagnosis.

Dipstick tests involve placing papers that contain small pads of chemicals into the urine sample. These papers, referred to as urine dipsticks and urine reagent strips, change color when exposed to various substances in the urine. This type of urine testing can result in false-positive or false-negative results. Therefore, most laboratories use urine dipstick controls to determine whether the dipsticks are performing properly.

Urine dipstick controls mimic patient samples and are fortified to target levels with compounds that produce the desired reaction when tested by the dipsticks. This is an accurate means of determining the performance of the dipsticks to insure accurate results from patient specimens. If a dipstick is found to provide inaccurate results, the dipsticks are discarded and a new package is tested. If the dipsticks in the new package perform within specifications, then the dipsticks are used to test actual patient samples. Medical technologists run controls on a daily basis in order to be compliant with state and federal regulations governing quality control in the clinical setting. The controls can be urine or synthetic urine.

Hospital and Reference Laboratories have used controls for urinalysis dipstick testing for many years. These laboratories typically have large refrigeration units located in close proximity to the actual location where testing is performed. This means that a medical technologist working in the lab would simply remove the control material from the refrigerator as needed. All liquid, ready-to-use, quality control material requires refrigeration in order to achieve maximum stability.

In recent years, a trend towards point-of-care urine dipstick testing has occurred. This means that more testing is being performed by nurses at the point-of-care. Nurses have different needs from medical technologists. One of the most important needs is that they do not have refrigeration suitable to store in-vitro diagnostic products, such as urine dipstick controls. The refrigeration that is available to them is usually dedicated for the storage of items such as medication and food. Therefore, nurses require controls that feature extended room temperature stability. Additionally, nurses require controls that are portable so that they can easily be transported to the point-of-care (bedside, nurses' station, etc.)

### SUMMARY

A control device that satisfies this need comprises an elongated package that has front and rear walls, a top portion and a bottom portion, and an elongated chamber substantially rectangular in vertical cross section. The chamber extends from the top portion to the bottom portion, and at least a portion of one of the walls of the chamber is substantially transparent or translucent so that positioning of a urine reagent strip in the chamber can be observed. The package includes a flap on one side of the chamber so that the package can be gripped without squeezing the chamber. Thus the flap is sufficiently large that the package can be held by the flap without any test urine passing out of the opened access section. The flap is at least 6.35 mm (one quarter inch) wide. There is test urine, which can be naturally occurring urine or synthetic urine, partially filling the chamber, and there is user information on at least one of the walls of the package. There is a closed access section in the top portion of the package, wherein when the access section is open, there is access to the chamber.

Thus, in a first aspect, the invention provides a control device suitable for a urine reagent strip comprising:
a) an elongated package comprising:
   1) front and rear walls,
   2) a top portion and a bottom portion,
   3) an elongated chamber substantially rectangular in vertical cross section extending from the top portion to the bottom portion, at least a portion of one of the walls at the chamber being substantially transparent or translucent,
   4) a flap at least 6.35 mm (¼ inch) wide on one side of the chamber, and
   5) a closed access section in the top portion, wherein when the access section is opened, there is access to the chamber;
b) test urine partially filling the chamber; and
c) user information on at least one of the walls,
wherein the flap is sufficiently large that the package can be held by the flap without any test urine passing out of the opened access section.

In a second aspect, the invention provides a control device suitable for a urine reagent strip comprising:
a) an elongated package comprising:
   1) front and rear walls,
   2) a top portion and a bottom portion,
   3) an elongated chamber substantially rectangular in vertical cross section extending from the top portion to the bottom portion, at least a portion of one of the walls at the chamber being substantially transparent or translucent, and
   4) a closed access section in the top portion, wherein when the access section is opened, there is access to the chamber;
b) test urine partially filling the chamber; and
c) user information on at least one of the walls,
wherein the front wall comprises, from the outside to the inside, a first layer selected from the group consisting of PET film and OPP film, a second layer selected from the group consisting of PVdC and EVOH, and a third layer selected from the group consisting of a film comprising LDPE and a film comprising LLDPE.

In a third aspect, the invention provides a control device suitable for a urine reagent strip comprising:
a) an elongated package comprising:
   1) front and rear walls,
   2) a top portion and a bottom portion,
   3) an elongated chamber substantially rectangular in vertical cross section extending from the top portion to the bottom portion,
   4) a closed access section in the top portion, wherein when the access section is opened, there is access to the chamber;
b) test urine partially filling the chamber; and
c) user information on the rear wall,
wherein a sufficiently large portion of the front wall is substantially transparent or translucent so that the amount the urine reagent strip is dipped into the chamber is visible through the front wall for ensuring the active portion contacts the test urine,
wherein when the access section is opened, there is sufficient access to the test chamber for dipping a urine reagent strip therein, and
wherein the chamber is at least 57 mm in length from the access section to the bottom of the chamber whereby a urine reagent strip, can be dipped therein with all of the active portion in contact with test urine.

The features of the foregoing devices may be combined in the same device.

The invention also provides a method for testing a urine reagent strip having active regions designed for contacting urine in a diagnostic test, the method comprising the steps of:
a. opening the access section of any of the devices defined above with the device oriented so the access portion is directed upwardly and so that test urine in the chamber does not leak out;
b. gripping the device, optionally by the flap; and
c. dipping the urine reagent strip a sufficient distance into the chamber so that all of the active regions of the urine reagent strip contact the test urine.

In a dip mode, preferably the open access section is sufficiently large and configured so that there is sufficient access for dipping the urine reagent strip therein. In a drip mode, the test urine can be dripped out of the chamber onto a regent strip, wherein the open access section need only be sufficiently large for placing drips of test urine on the reagent strip. The access section can comprise a weakened portion in the walls. The test urine is stable at room temperature for at least 30 days, preferably at least 60 days, and most preferably at least 90 days.

Preferably the flap extends substantially the entire length of the package, and more preferably said flap is on both sides of the chamber.

Preferably in the dip configuration of the invention the chamber is sufficiently long that a urine reagent strip can be dipped therein with all active portions of the urine reagent strip in contact with the test urine. Most typically the chamber is at least 9 cm in length from the access section to the bottom of the chamber.

Typically the chamber is at least about 5 cm in length, with a maximum length of about 12.5 cm. Typically the chamber has a width of about 0.75 to about 1.5 cm.

The device is a single use device, where the volume of the chamber in the dip configuration is sufficiently small the urine reagent strip can be dipped therein without any test urine leaking out of the chamber. Most typically the chamber contains test urine in the amount of no more than about 80% of the total capacity of the chamber. The amount of test urine is typically less than 5 ml.

Preferably for easy placement of the strip in the chamber, at least one of the walls at the chamber is substantially transparent or translucent the entire length of the chamber.

Typically the front and rear walls are formed of different materials, and the portions of the front and rear walls in contact with the test urine are substantially non-reactive with the test urine. In addition preferably the portions of the front and rear walls forming the chamber are substantially impermeable to oxygen so that the test urine is not degraded.

In use of the device the access section is opened with the device oriented so that the access section is directed upwardly so that test urine in the chamber does not leak out. The device is gripped by the flap and, in the dip mode, the urine reagent strip is dipped into the chamber a sufficient distance so that all the active regions of the urine reagent strip contact the test urine. In the drip mode, test urine is dripped out the chamber onto the reagent strip, such as by squeezing the chamber.

This device, which addresses the unique requirements of point-of-care urine dipstick quality control, can be used to monitor the performance of visual and instrumental readings or urine dipsticks by immersing the dipstick into the control - the same way a patient sample is tested. The test urine preferably includes values for all urine dipstick analytes plus microalbumin and creatinine; and qualitative results for hCG early pregnancy detection test methods. The control is also suitable for use with confirmatory tests and refractometry.

The test urine, also referred to as a control is thus packaged in a unitized, device that is discarded after use. Multiple devices can be packaged in boxes containing approximately one hundred devices. The control preferably is stable at refrigerator temperature for approximately two years. Once removed from the refrigerator, the control is stable for at least thirty days, preferably at least sixty days, and more preferably at least ninety days at room temperature until opened.

### DRAWINGS

These and other features, aspects, and advantages of the present invention will become better understood with reference to the following description, appended claims, and accompanying drawings where:
Figure 1 is a front plan view of the front of a product having features of the present invention and comprising a plurality of unitized plastic urine dipstick control devices;
Figure 2 is a rear plan view of the product of Figure 1;
Figure 3 is an elevational view of a urine dipstick to be inserted into one of the packages of the product of Figures 1;
Figure 4 is a vertical sectional view of the device of Figure 1 taken on line 4-4 in Figure 2;
Figure 5 is a front plan view of a second version of the invention for use for dripping or dropping test urine onto a reagent strip; and
Figure 6 is a front plan view of one of the devices of Figure 1 presenting preferred dimensions for the device.

### DESCRIPTION

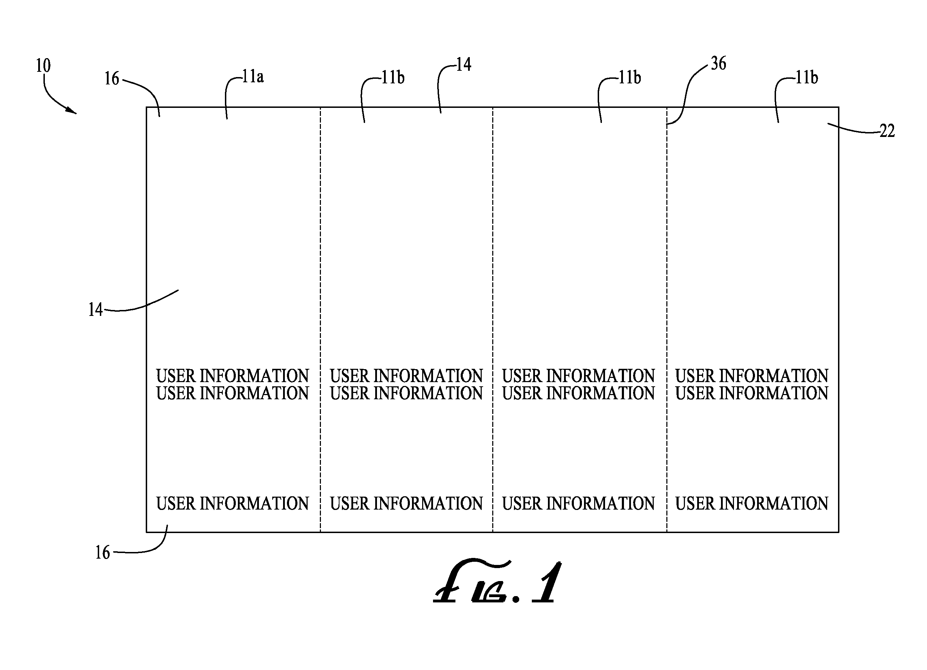

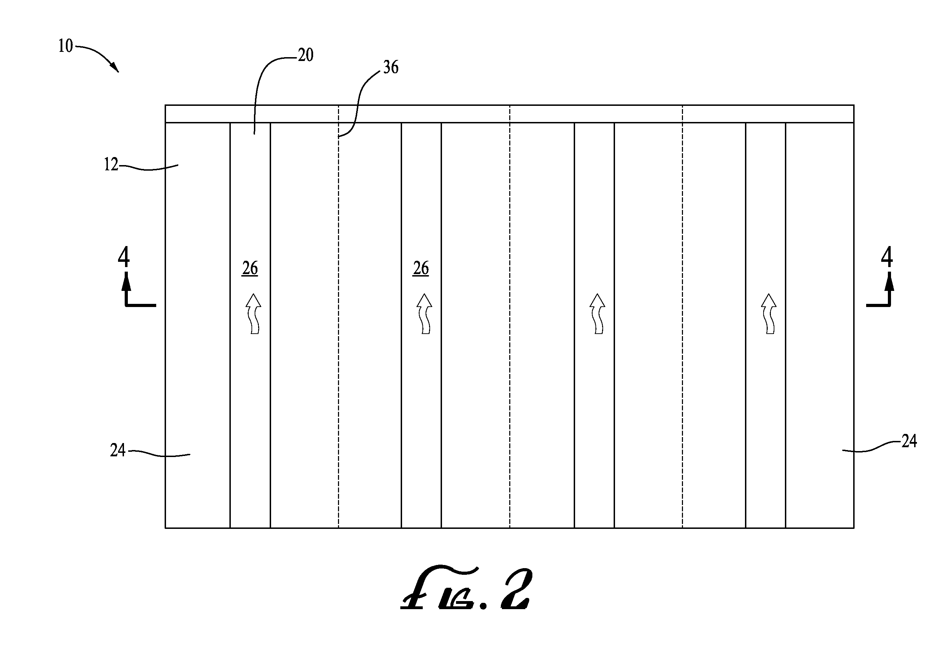

A packaged product 10 in Figure 1 comprises four elongated urine control devices 11a-d. Each device 11 can be formed by the linking, such as by fusion or adhesive of a (i) clear, namely substantially transparent or translucent, plastics material rear wall 12 (also referred to as a layer) (Figure 2) and a (ii) front foil wall 14 (Figure 1) (also referred to as a layer). Each device 11 has a top portion 16, a bottom portion 18, and an elongated chamber 20 substantially rectangular in vertical cross section extending from the top portion 16 to the bottom portion 18. There is a closed access section 22 in the top portion 16, wherein when the access section 22 is opened, there is access to the chamber 20. There is at least one flap 24 at on one side of the chamber 20, and preferably on both sides. There is test urine 26 partially filling the chamber 20, and user information on at least one of the walls. Each flap 24 is sufficiently large that the device 11 can be held by one of the flaps 24 without any test urine 26 passing out of the opened access section.

Generally each of the flaps 24 extends substantially the entire length of the device 11. The flaps 24 are wide sealed sections. The flaps 24 are at least 6.25 mm (one quarter inch) wide, ideally on both sides of the chamber 20, so that the device can be held by hand without pressing on the chamber 20. Pressure on the chamber could result in contents of the chamber 20 being inadvertently dispersed.

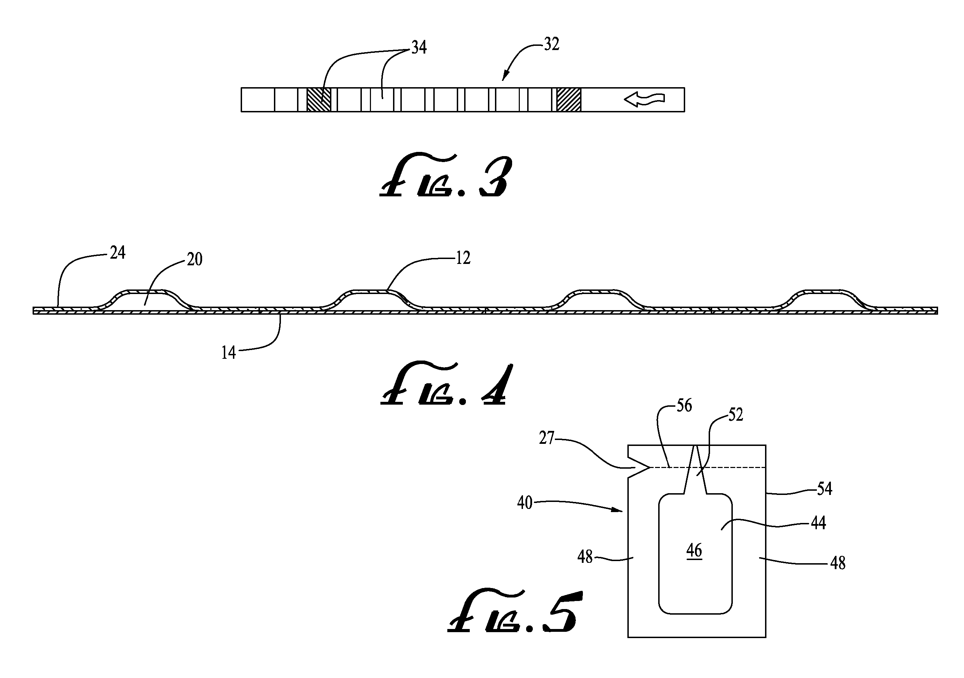

In the version of the invention shown in Figure 1, referred to as a "dip" version, the chamber 20, which has a flexible, clear plastic side 12, accommodates a urine dipstick 32 (see Figure 3) having a plurality of spaced pads 34 with precision tolerances, allowing for maximum control of the saturation of each pad 34. The outer portion of the foil side 14 (Figure 1) is used to print user information such as the product name and other necessary information, such as instructions, date packaged, and other FDA required information. Each device contains a predetermined amount of control fluid, namely test urine. The term "test urine" refers to both naturally occurring urine and synthetic urine.

The product 10 can be delivered as a single unit, or with approximately four or five devices 11 per sheet (or in a roll) as seen in Figure 1, depending on the rigidity of the final plastics material. Each device 11 can be torn from the sheet, or roll, as needed, along tear lines 36. Once separated, the device must 11 is oriented vertically in order to allow the control fluid to settle to the bottom of the chamber 20. This is visible through the clear, plastics material side (Figure 2) 12 of the device. Optionally the device can be opened by slightly peeling apart the plastic side from the foil side at the top of the device, i.e. at the access section 20, as indicated by the legend Tear Here or other indicia (Figure 2). The device 11 is designed to allow a predetermined amount of separation, enough to introduce the urine dipstick 32, but not enough to over separate plastic from foil.

In the dip version of the invention, the volume of test urine in the chamber is sufficiently small that the urine reagent strip can be dipped therein without any test urine leaking out of the chamber. For example, the chamber can contain test urine amount in an amount no more than about 80% of the total capacity of the chamber.

Once adequate separation is achieved, the urine dipstick, such as the dipstick 32 (Figure 3), can be introduced into the chamber 20. As the dipstick 32 is immersed into the control fluid, the fluid level rises - saturating each pad 34 with control fluid. The tolerances in the sleeve provide maximum control as to which pads are immersed. This is important because some dipsticks recommend against flooding the last pad on the dipstick 32.

Once satisfactory immersion has been achieved, the dipstick 32 is removed from the device and the device is discarded.

Exemplary of commercial reagents strips for urine analysis is Siemens Mutlistix™ 10 SG which tests for glucose, bilirubin, ketone, specific gravity, blood, pH, protein, urobilinogen, nitrite, and leukocytes. The Siemens strip is about 14.6 cm (5 3/4 inches) long, about 0.5 cm (0.2 inch) wide, about 0.1 cm (0.04 inch) thick at the pads 34, with the portion of the strip needing contact with the test urine being about 8.1 cm (4 3/16 inches) long.

The base or bottom portion 18 of each device 11 can be flared in such a way that it will allow the device 11 to stand on its own in the upright position when placed on a flat, level surface. This allows the user to set the device down after opening, if desired, so that the control fluid does not leak from the device.

It is desirable that the materials used for forming the device 11, at least in the region of the chamber, be water proof, non reactive with all the constituents of the test urine, and substantially oxygen and water vapor impermeable so the test urine has at least a thirty day useful life without refrigeration. Due to conflicting demands of transparency and printability, generally the front and rear walls are made of different materials. Preferably any portions of the walls in contact with the test urine are substantially non-reactive with the test urine.

The device 11 is formed of two layers sealed together, where at least the rear layer 12 in the region of the chamber is substantially transparent or translucent so the contents of the chamber 20 can be seen, particularly as the dip stick 32 is inserted into the chamber 20. At least one of the layers needs to be printable. Also preferably the access section 28 is openable without the use of a tool. A notch, such as notch 27 in combination with a laser score 56 as shown in Figure 5, or easily opened seal can be provided.

Generally the test urine is provided in two versions, namely urine providing normal levels of the constituents mark, so that all the markers on a reagent strip, if the reagent strip is operating properly, reports normal levels. Alternatively the test urine can be what is called level 2, where all the markers reveal positive on a urine reagent strip that is operating normally.

When reference is made in this application to human urine or naturally occurring urine, this is meant that human urine is used as a starting liquid. Generally the urine is modified to provide either normal levels of the various constituents for which testing is being conducted, or alternatively, adjusted to level 2.

For long term stability, preferably the walls resist oxygen and water vapor transmission. Preferably the oxygen and moisture barrier ranges are:
OTR (oxygen transmission rate) - <0.02 cc/100 in2/24 hours @73°F (23°C), 0% RH
WVTR (water vapor transmission rate) - <0.16 g/100 in2/24 hours @ 100°F (38°C), 90% RH

With reference to Figure 5, a "drop" device 42 having features of the present invention comprises a central chamber 44, typically containing 3 ml or less of test urine 46, with a flap 48 of at least one quarter inch size on each side of the chamber. At the top of the chamber is a nozzle shaped outlet 52 in an access section 54, which is opened by tearing perforations 51 using the notch 27 as a guide for where tearing should occur. Less test urine is needed for the drop version than the dip version of the invention because test urine need only to be dropped active regions of the dip stick 32.

In the dip version of the invention, the volume of test urine in the chamber is sufficiently small that the urine reagent strip can be dipped therein without any test urine leaking out of the chamber. For example, the chamber can contain a test urine amount in an amount no more than about 80% of the total capacity of the chamber.

The devices 11 and 40 can be formed from two separate film webs brought together and heat sealed on four sides. Due to the functional requirements of the device typically the materials used are multi-layer structures. Test urine compatibility, heat sealable, excellent oxygen barrier and moisture barrier, clear back panel, and printed front panel are all desirable features of the device. Each layer of a multi-layer film is the chosen to provide one or more of these attributes. Film layer materials that can be used to make the device are listed in the Table 1.

**TABLE 1**

| **Layer** | **Desired Features** | **Materials** |
|---|---|---|
| Outer | Strength, dimensional stability, printable, heat stability | PET Film, OPP film |
| Middle | Oxygen barrier, and with the exception of EVOH, also moisture barrier | Aluminum foil, PVdC film or coating, EVOH film, SiO₂(by plasma deposition), Al₂O₃ (by sputtering) |
| Laminating Adhesive | Bonds layers together | Solvent-based - acrylic or polyurethane Water-based - acrylic emulsions and polyurethane dispersions Solventless types - 2-part polyurethane, 2-part epoxy |
| Inner | Heat seal, product contact surface, moisture barrier | LDPE film, LLDPE film, EVA (added to LDPE or LLDPE), EAA (coextruded with LDPE or LLDPE) |

PET is polyethylene teraphthalate
OPP is oriented polypropylene
PVdC is polyvinylidene chloride
EVOH is ethylene vinyl alcohol polymer
LDPE is low density polyethylene
LLDPE is linear low density polyethylene
EVA is ethylene vinyl acetate
EAA is ethylene acrylic acid

Table 2 list multi-layer laminated film structures that can be used for forming the front wall 14 of the device, where the front is an opaque film and the back is clear film.

### TABLE 2

Device Front Opaque Film Structures - (outside layer to inside layer)
1. 48ga. PET / Ink / adhesive / 0.00028" foil / adhesive / 1.0 mil LDPE film
2. 50ga. OPP / Ink / 7# white LDPE / adhesive / 0.000275" foil / adhesive /2.5# EAA-15.5# LDPE coextruded
3. 50ga. OPP /Ink / 7# white LDPE-EAA coextruded / adhesive/0.000255" foil / adhesive / 2.5# EAA-15.5# LDPE coextruded
4. 48ga. PET / Ink / adhesive / 0.000285" foil 1100-0 / adhesive / 2.0 mil sealant layer metallocene LLDPE and EVA blend (10 - 30% by weight EVA) - 3mil overall thickness

Notes:
Ga = gauge; 50 ga = .0005 inch
Printing is performed with ink on the reverse side of the outer film
# = weight per ream, so 7# = 7 pounds per ream

Table 3 list multi-layer laminated film structures that can be used for forming the rear wall 12 of the device, where the rear wall is substantially transparent.

### TABLE 3

Device Back Clear Film structures - (outside layer to inside layer), ink is included for printed structures only.
1. 50 ga. OPP / Ink / adhesive / 3# PVDC / 7# LDPE / adhesive / 1.0 mil LDPE
2. 50 ga. OPP / Ink / adhesive / 3# PVDC / 7# LDPE / adhesive /1.5 mil LDPE
3. 50 ga. OPP / Ink / adhesive / 5# PVDC / 7# LDPE / adhesive / 1.5 mil LDPE
4. 35 ga. PET / adhesive / 1.5 mil EVOH-LDPE coextruded
5. 32 ga. PET / adhesive/ 1.5 mil EVOH-LDPE coextruded
6. 70 ga. OPP / adhesive / 1.5 mil LLDPE-EVOH-LDPE coextruded
7. 50 ga. OPP / Ink / adhesive / EVOH / adhesive / 50 ga. OPP / adhesive / 1.0 mil LDPE
8. 48 ga. PET (SiO₂) / adh / 1 mil LDPE
9. 48 ga. PET (SiO₂) / 1 mil LDPE
10. 48 ga. PET (Al₂O₃) / adhesive / 1 mil LDPE
11. 48 ga. PET ((Al₂O₃) / 1 mil LDPE

Conventional techniques are used to form the laminated films. For example, conventional coextrusion techniques, and lamination with heat, pressure and/or adhesive can be used. As a specific example, film 2 of Table 2 can be formed by printing the back side of the OPP layer, extrude the LDPE onto OPP film, coextrude the EAA with the LDPE, and adhesive laminate the two resulting structures together with the foil in between. This can be heat sealed to film 4 of Table 3 while filling the chamber with test urine.

Preferably the entire front wall is clear and the rear wall is opaque white with printing thereon providing user information.

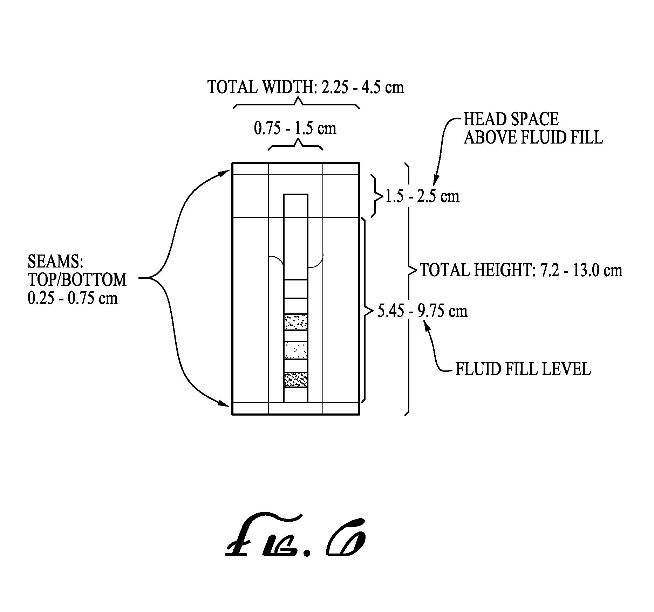

Figure 6 shows typical dimensions of a preferred device that accommodates dipping by conventional reagent strips. Thus preferably the chamber is from about 5.7 to about 12.5 cm in length and from about 0.75 to about 1.5 cm in width.

### Example 1

### Dip version invention.

Dip device 11 is 11.3 cm tall and 3 cm wide, sealed on both sides and has a central 1 cm wide chamber central containing test urine. The chamber has 9.3 cm of test urine with 2 cm head space. The volume of the liquid in the chamber is about 4.8 cubic centimeters. The flap on both sides of the chamber is 1 cm wide. The front wall is made of material 2 from Table 2 and the rear wall is made of material 4 from Table 3. The top and bottom seals are 5 to 7 mm wide. The device has printing on the front wall. The device has a notch placed in one side seal, such as shown in Figure 5, to assist initiation of the tear opening across the top of the chamber along a tear line. A laser score, such as shown in Figure 5 is placed on the front wall to insure the tear occurs straight along the top of the device. The laser score is registered to the print and is located at the top of the device during chamber formation and filling. The device is made and filled on a vertical-form-fill-seal machine (VFFS).

### Example 2

### Drop version invention.

With reference to Figure 5, a drop device 40 is about 6 cm tall with a chamber 3 cm in height and 2 cm in width, containing about 2 ml of test urine, with a one quarter inch flap on each side of the chamber. The materials used for forming the device are the same as for Example 1. The device 40 has printing on the front wall. The device has a notch placed in one side seal to assist initiation of the tear opening across the top of the chamber. A laser score is placed on the front wall to insure the tear occurs straight along the top of the device. The laser score is registered to the print and is located at the top of the device during chamber formation and filling. The device is made and filled on a vertical-form-fill-seal machine (VFFS).

Although the present invention is described in considerable detail with reference to certain preferred versions thereof, other versions are possible. For example, a stand-up device, preferably with at least ¼ inch wide flap, can have a bottom gusset formed into the device with the sides of the gusseted area sealed together. The center unsealed pouch bottom section can be expanded outward on both sides to form a base on which the pouch can stand upright. This type of pouch can be formed from a single web on a horizontal machine and is either filled on the same machine or filled on a different machine in a separate process. If it is made from a single web, the same material is used for the front and rear walls. Therefore the subject claims should not be limited to the preferred versions described.

All the features disclosed in this specification (including any accompanying claims, abstract, and drawings) may be replaced by alternative features serving the same, equivalent or similar purpose, unless each feature disclosed is one example only of a generic series of equivalent or similar features.

## Claims

1. A control device suitable for a urine reagent strip comprising:
a) an elongated package comprising:
1) front and rear walls,
2) a top portion and a bottom portion,
3) an elongated chamber substantially rectangular in vertical cross section extending from the top portion to the bottom portion, at least a portion of one of the walls at the chamber being substantially transparent or translucent,
4) a flap at least 6.35 mm (¼ inch) wide on one side of the chamber, and
5) a closed access section in the top portion, wherein when the access section is opened, there is access to the chamber;
b) test urine partially filling the chamber; and
c) user information on at least one of the walls,
wherein the flap is sufficiently large that the package can be held by the flap without any test urine passing out of the opened access section.

2. The device of claim 1 wherein when the access section is opened, there is sufficient access to the test chamber for dipping a urine reagent strip therein.

3. The device of claim 1 or 2 comprising said flap on both sides of the chamber.

4. The device of claim 1, 2 or 3 wherein the or each flap extends substantially the entire length of the package.

5. The device of any preceding claim wherein the chamber is sufficiently long that a urine reagent strip that is 146 mm (5 3/4 inches) long, 5 mm (0.2 inch) wide, and 1 mm (0.04 inch) thick where it has active portions, with the portion of the strip needing contact with the test urine being 81 mm (4 3/16 inches) long, can be dipped in the chamber with all of the active portions of the urine reagent strip in contact with test urine, and, optionally, wherein the chamber is at least 90 mm in length from the access section to the bottom of the chamber.

6. The device of claim 5 wherein the volume of test urine in the chamber is sufficiently small that the urine reagent strip can be dipped therein without any test urine leaking out of the chamber, and, optionally, wherein the chamber contains test urine in an amount of no more than about 80% the total capacity of the chamber.

7. The device of any preceding claim
wherein at least one of the walls at the chamber is substantially transparent or translucent the entire length of the chamber; and/or
wherein the front and real walls are formed of different materials; and/or wherein the portions of the front and rear walls in contact with the test urine are substantially non-reactive with the test urine; and/or
wherein the portions of the front and rear walls forming the chamber are substantially impermeable to oxygen; and/or
wherein the access section comprises a weakened portion in the walls; and/or containing less than 5 mL of test urine; and/or
wherein the test urine is natural urine or synthetic urine.

8. The device of any preceding claim formed by securing material 2 of Table 2 to the material 4 of Table 3, and partially filling the chamber with the test urine.

9. A control device suitable for a urine reagent strip comprising:
a) an elongated package comprising:
1) front and rear walls,
2) a top portion and a bottom portion,
3) an elongated chamber substantially rectangular in vertical cross section extending from the top portion to the bottom portion, at least a portion of one of the walls at the chamber being substantially transparent or translucent, and
4) a closed access section in the top portion, wherein when the access section is opened, there is access to the chamber;
b) test urine partially filling the chamber; and
c) user information on at least one of the walls,
wherein the front wall comprises, from the outside to the inside, a first layer selected from the group consisting of PET film and OPP film, a second layer selected from the group consisting of PVdC and EVOH, and a third layer selected from the group consisting of a film comprising LDPE and a film comprising LLDPE.

10. The device of claim 9 wherein the rear wall comprises, from the outside to the inside, a first layer selected from the group consisting of PET film and OPP film, and a second layer selected from the group consisting of a film comprising LDPE and a film comprising LLDPE.

11. The device of claim 9 or 10 having the features of any of claims 1 to 8.

12. A control device suitable for dipping a urine reagent strip therein, the urine reagent strip having an active portion, the device comprising:
a) an elongated package comprising:
1) front and rear walls,
2) a top portion and a bottom portion,
3) an elongated chamber substantially rectangular in vertical cross section extending from the top portion to the bottom portion,
4) a closed access section in the top portion, wherein when the access section is opened, there is access to the chamber;
b) test urine partially filling the chamber; and
c) user information on the rear wall,
wherein a sufficiently large portion of the front wall is substantially transparent or translucent so that the amount the urine reagent strip is dipped into the chamber is visible through the front wall for ensuring the active portion contacts the test urine,
wherein when the access section is opened, there is sufficient access to the test chamber for dipping a urine reagent strip therein, and
wherein the chamber is at least 57 mm in length from the access section to the bottom of the chamber whereby a urine reagent strip, can be dipped therein with all of the active portion in contact with test urine.

13. The device of claim 12,
wherein the length of the chamber is from about 57 to about 125 mm; and/or. wherein the chamber is from about 0.75 to about 1.5 cm in width.

14. The device of claim 12 or 13 having the features of any of claims 1 to 11.

15. A method for testing a urine reagent strip having active regions designed for contacting urine in a diagnostic test, the method comprising the steps of:
a. opening the access section of the device of any preceding claim with the device oriented so the access portion is directed upwardly and so that test urine in the chamber does not leak out;
b. gripping the device, optionally by the flap; and
c. dipping the urine reagent strip a sufficient distance into the chamber so that all of the active regions of the urine reagent strip contact the test urine.
